# EUROPEAN PATENT APPLICATION

(11) **EP 4 029 524 A1**
(43) Date of publication of application: **20.07.2022**
(21) Application number: 21151294.2
(22) Date of filing: 13.01.2021
(51) Int. Cl.: A61L 2/00, A61L 2/10, A61L 2/24, E05B 1/00, E03D 9/00, A47K 13/00

(54) **APPLYING A SAFETY ALGORITHM IN A SYSTEM COMPRISING A RADIATION BODY AND AT LEAST ONE RADIATION SOURCE**

(71) Applicant: Koninklijke Philips N.V., 5656 AG Eindhoven (NL)
(72) Inventor: Hietbrink, Roelant Boudewijn, 5656 AE Eindhoven (NL); Salters, Bart Andre, 5656 AE Eindhoven (NL); NIESSEN, Eduard Matheus Johannes, 5656 AE Eindhoven (NL); Martens, Peter, 5656 AE Eindhoven (NL)
(74) Representative: Philips Intellectual Property & Standards

(57) **Abstract**

In a system (1) comprising a radiation body (10), a radiation source (20) configured to emit radiation (21) at least comprising UV radiation, a detector (30) configured to detect an internal radiation intensity in the radiation body (10), and a controller arrangement (40), the controller arrangement (40) is configured to apply a safety algorithm after occurrence of a disturbance incident caused by an object (2) contacting a radiation exit window (11) of the radiation body (10). The safety algorithm involves setting an evaluation state of the radiation source (20) during evaluation periods separated by evaluation intervals while otherwise setting an adapted state of the radiation source (20), and determining a value of the internal radiation intensity during the evaluation periods in order to assess whether it is safe to restore the normal state of the radiation source (20).

## Description

### FIELD OF THE INVENTION

The invention relates to a system comprising a radiation body comprising a radiation exit window, wherein the radiation body is configured to receive radiation that at least comprises UV radiation, and wherein the radiation exit window is capable of allowing at least part of the radiation to pass to the exterior of the radiation body; at least one radiation source configured to provide the radiation; at least one detector configured to detect an internal radiation intensity in the radiation body; and a controller arrangement, functionally coupled to the at least one radiation source and the at least one detector.

Further, the invention relates to an object comprising the system as described here before, wherein the radiation exit window of the radiation body of the system is arranged to be at an exterior side of the object.

Still further, the invention relates to a method of adding a system as described here before to an existing object, wherein the radiation body of the system is applied to an exterior surface of the existing object.

### BACKGROUND OF THE INVENTION

WO 2018/215272 A1 discloses a system comprising a waveguide element, an optical sensor and a control system. The waveguide element comprises a radiation exit window, wherein the waveguide element is i) configured to receive radiation, wherein the radiation at least comprises UV radiation, ii) configured to radiate at least part of the radiation to the exterior of the waveguide element via the radiation exit window, and iii) configured to internally reflect part of the radiation at the radiation exit window. The optical sensor is configured to sense an internal reflection intensity of the internally reflected radiation. The control system is functionally coupled to the optical sensor and is configured to reduce the intensity of the radiation as function of reaching a predetermined first threshold of a reduction of the internal reflection intensity over time.

WO 2018/215272 A1 teaches that the UV radiation is used for killing microorganisms as may be present on the radiation exit window, or for rendering the microorganisms inactive or unable to reproduce. Practical examples of microorganisms are bacteria. The disinfecting effect of using UV radiation is mainly governed by a total dose of the UV radiation. In the context of using UV radiation, it may be necessary to take specific measures when higher organisms, including human beings, may be in a position of receiving the UV radiation, which is especially the case if it is possible for the higher organisms to physically contact radiation emitting surfaces.

During operation of the known system, radiation is coupled out of the waveguide element through the radiation exit window if some object touches the window. This outcoupling means that less radiation will stay inside the waveguide element and is also referred to as frustration of the (total) internal reflection. Therefore, by using the optical sensor to monitor an internal reflection intensity of the internally reflected radiation, it is possible to detect a situation of some object touching the window, especially some object that is considerably larger than microorganisms, such as a human hand or finger. When a considerable step is observed in the signal provided by the optical sensor, it is assumed that this means that a relatively large object contacts the window. In order to ensure safety in such a situation, it may be determined that the radiation needs to be shut off, at least temporarily.

Among other things, the invention that is the subject of WO 2018/215272 A1 provides an object comprising the system, wherein the object comprises an external surface, and wherein the radiation exit window of the waveguide element of the system is configured as at least part of the external surface. Examples of such an object include a door knob, a tap knob, a toilet knob, a seating of a toilet, a railing, a kitchen cutting board, a kitchen wall, a table, or (other) common (household) objects, i.e. objects which are especially designed to be used at home or in offices, etc. Further examples of such an object include a cleanroom wall and medical devices such as an operating table and an operation room wall. In the context of all of such possible practical applications of the invention that is the subject of WO 2018/215272 A1, it is important that external surfaces are kept in a disinfected state while situations in which the UV radiation used in the process might cause harm to higher organisms such as human beings need to be avoided.

Generally speaking, applying a system such as known from WO 2018/215272 A1 is beneficial in a situation in which disinfection of surfaces may contribute to avoiding contamination. Such a situation can occur in all kinds of environments, including public spaces, work environments and domestic settings. For example, if no disinfecting measures are applied, control buttons and touchscreens in public spaces constitute spots which involve a health risk, because transfer of infectious diseases may take place through the control buttons and touchscreens. Bacteria and viruses may be left on a control button or touchscreen by a first person, and subsequently get picked up by a next person, whereby a disease carried by the first person is spread. Public use of control buttons and touchscreens is common in view of the fact that control buttons and touchscreens are found in many types of devices such as elevators, ATM machines, order terminals, payment terminals and vending machines.

As explained in the foregoing, the system known from WO 2018/215272 A1 is effective in disinfection of surfaces, because radiation is coupled out of the waveguide element at the very positions on the waveguide element where there is any form of contamination such as virus or bacteria particles. However, the same principle of outcoupling the radiation at contact positions would cause potentially unsafe exposure of human beings touching the waveguide element to UV radiation if it was not for the measure of detecting the touching and shutting off the radiation. In the context of the present invention, it is acknowledged that there is a need for developing useful ways of actually putting this measure to practice.

### SUMMARY OF THE INVENTION

It is an object of the invention to design a system comprising a radiation body and at least one radiation source in such a way that a proper balance is realized between effectively obtaining disinfection results at the position of a radiation exit window of the radiation body on the one hand and protecting human beings and other higher organisms from harmful exposure to UV radiation provided by the at least one radiation source on the other hand. In the context of the invention, the radiation body does not necessarily need to be the same as the waveguide element disclosed in WO 2018/215272 A1, particularly does not necessarily need to be configured to have a radiation guiding function and to rely on the (total) internal reflection phenomenon in that respect, although the invention does cover the use of such a waveguide element.

In view of the foregoing, the invention provides a system comprising a radiation body comprising a radiation exit window, wherein the radiation body is configured to receive radiation that at least comprises UV radiation, and wherein the radiation exit window is capable of allowing at least part of the radiation to pass to the exterior of the radiation body; at least one radiation source configured to provide the radiation; at least one detector configured to detect an internal radiation intensity in the radiation body; and a controller arrangement, functionally coupled to the at least one radiation source and the at least one detector, configured to set a normal state of the at least one radiation source in which the at least one radiation source provides the radiation with an intensity at an operational level as a default, and to set an adapted state of the at least one radiation source in which the at least one radiation source provides the radiation with an intensity at an adapted level that is reduced relative to the operational level after occurrence of a disturbance incident involving a change of the internal radiation intensity detected by the at least one detector, and configured to apply a safety algorithm after occurrence of a disturbance incident that involves setting an evaluation state of the at least one radiation source during evaluation periods separated by evaluation intervals, in which evaluation state the at least one radiation source provides the radiation with an intensity at an evaluation level that is increased relative to the adapted level, and setting the adapted state of the at least one radiation source outside of the evaluation periods, and that involves determining a value of the internal radiation intensity during the evaluation periods, and to abort the safety algorithm and to restore the normal state of the at least one radiation source when the value of the internal radiation intensity is inside an evaluation reference range of values.

It follows from the foregoing that the system according to the invention comprises i) a radiation body comprising a radiation exit window, wherein the radiation body is configured to receive radiation that at least comprises UV radiation, and wherein the radiation exit window is capable of allowing at least part of the radiation to pass to the exterior of the radiation body, ii) at least one radiation source configured to provide the radiation, iii) at least one detector configured to detect an internal radiation intensity in the radiation body, and iv) a controller arrangement, functionally coupled to the at least one radiation source and the at least one detector. The controller arrangement is configured to set a normal state of the at least one radiation source as a default, and to set an adapted state of the at least one radiation source after occurrence of a disturbance incident. In this way, assuming that occurrence of a disturbance incident is indicative of the radiation exit window being contacted by a relatively large object such as a human hand or finger, the controller arrangement is configured to enable disinfection of the radiation exit window as a default, and to implement a safety measure of reducing the intensity at which radiation is provided whenever appropriate. The adapted level of the intensity of the radiation provided by the at least one radiation source in the adapted state may be a zero level, although this is not necessary in the context of the invention.

According to a notable aspect of the invention, the controller arrangement is configured to apply a safety algorithm after occurrence of a disturbance incident. The safety algorithm provides an iterative way of assessing whether or not circumstances prevailing due to the disturbance incident are still present, so as to determine if the adapted state of the at least one radiation source needs to be maintained or if the normal state of the at least one radiation source can be restored. To that end, the safety algorithm involves setting an evaluation state of the at least one radiation source during evaluation periods separated by evaluation intervals, and setting the adapted state of the at least one radiation source outside of the evaluation periods, and further involves determining a value of the internal radiation intensity during the evaluation periods, wherein the controller arrangement is configured to abort the safety algorithm and to restore the normal state of the at least one radiation source when the value of the internal radiation intensity is inside an evaluation reference range of values. Thus, an effective and safe way of operating the system is realized, wherein the normal state of the at least one radiation source is set as a default, wherein the adapted state of the at least one radiation source is set after occurrence of a disturbance incident, and wherein the adapted state of the at least one radiation source is maintained only as long as the disturbed circumstances last. By iteratively assessing whether the normal state of the at least one radiation source can be restored and doing so when evaluation demonstrates that the value of the internal radiation intensity is the evaluation reference range of values in an evaluation period, it is achieved that the time that the system can be effective in performing a disinfection action is optimized, wherein it is especially advantageous that the disinfection action can be resumed directly after the cause of the disturbance incident has been removed. Also, it is an advantage of the invention that applying the safety algorithm can be done without a need for additional components in the system, so that there is no increase of complexity nor cost of the system.

According to a feasible option, occurrence of a disturbance incident is determined when it appears that a value of the internal radiation intensity is outside of a safety reference range of values and/or a change of the internal radiation intensity in predetermined direction takes place at a rate that is higher than a disturbance reference rate. The invention covers various options which may be at the basis of a change of the internal radiation intensity and which are related to the constitution of the system, particularly the features of the radiation body, including the option of such a change resulting from an increase of radiation exiting the radiation body, the option of such a change resulting from an increase of radiation being reflected into the radiation body, and the option of such a change resulting from a combination of a change of exiting radiation and a change of incoming radiation.

In an advantageous embodiment of the system according to the invention, the controller arrangement is configured to enable adjustment of the safety reference range of values. In this respect, according to one feasible option, the safety algorithm includes a step of adjusting the safety reference range of values on the basis of a preceding value of the internal radiation intensity detected during a period directly prior to occurrence of a disturbance incident so as to include the preceding value and a tolerance window. In that way, it can be guaranteed that the safety reference range of values is representative of an actual constitution of the system at the very time of the disturbance incident. Alternatively, according to another feasible option, the controller arrangement is configured to apply a calibration algorithm that involves determining a calibration value of the internal radiation intensity during calibration periods separated by calibration intervals, and that involves adjusting the safety reference range of values on the basis of the calibration value of the internal radiation intensity so as to include the calibration value and a tolerance window. The calibration intervals may be of equal duration and may be 24-hour intervals, for example, and, depending on the practical application of a system, the time at which the calibration algorithm is executed is preferably set to be a time at which the chance of the radiation exit window getting contacted by a higher organism such as a human being is minimal to zero. In a large number of feasible applications of the system, this may imply that a time such as 3 am may be appropriate for executing the calibration algorithm, for example.

Another or additional way of adjusting the safety reference range of values that is covered by the invention involves having a user interface for receiving user input to that end. Further, it is to be noted that the invention also relates to an option of the safety reference range of values being a fixed system setting. In any case, information representing reference ranges and/or reference rates can be stored in a database or the like, and the safety algorithm can include a step of retrieving such information from the database or the like. Although it is advantageous to have automatic calibration, the invention also covers an option of the controller arrangement being configured to apply a calibration algorithm that involves determining a calibration value of the internal radiation intensity during calibration periods initiated on the basis of user input.

In respect of the safety algorithm and particularly the duration of the evaluation intervals, it is noted that it may be advantageous if the safety algorithm includes increasingly longer duration of successive evaluation intervals. For example, in the context of a doorknob, a first evaluation interval may last 1 second. If the evaluation demonstrates that the normal state of the at least one radiation source cannot be restored at that point, one or more further evaluation intervals of longer duration can be applied, wherein the first evaluation interval is appropriate in view of a normal situation in which the doorknob is touched for only a short time, and wherein the need for further evaluations indicates a less common situation of the doorknob being held for a longer time. Also, the invention offers the possibility of choosing the duration of evaluation intervals in dependence on the intended application of the system according to the invention. For example, the evaluation intervals may generally be shorter in the context of a button of a coffee machine than in the context of a button of an elevator, assuming that people walk away from a coffee machine after having retrieved their coffee and that people stay close to an elevator's control panel for some time.

In order to ensure safety of human beings or other higher organisms touching the radiation exit window of the radiation body, under all circumstances, also during the evaluation periods, it is advantageous if each of the evaluation periods is at least a thousand times shorter than the evaluation interval preceding the evaluation period. In the example of the evaluation interval taking 1 second, this implies a duration of the evaluation period of only 1 millisecond. Having an increased level of the intensity of the UV radiation during such a short time does not pose a health risk in any way.

The evaluation level of the intensity of the radiation provided by the at least one radiation source in the evaluation state may be the operational level, which does not alter the fact that it is also possible that the evaluation level of the intensity of the radiation provided by the at least one radiation source in the evaluation state is between the adapted level and the operational level. The latter may be preferred from a safety point of view, as it may happen that the radiation exit window is still touched during one or more evaluation periods. If the evaluation level of the intensity of the radiation provided by the at least one radiation source in the evaluation state is the operational level, and the controller arrangement is configured to apply a safety reference range of values in the process of determining occurrence of a disturbance incident, as suggested earlier, the evaluation reference range of values may be the safety reference range of values.

The number of radiation sources and the number of detectors in the system can be chosen freely. Depending on the constitution of the radiation body and the size of the radiation exit window, it may be advantageous if the system comprises at least two radiation sources arranged in the radiation body at a distance from each other and/or at least two detectors arranged in the radiation body at a distance from each other. Also, it is possible to have more than one detector at one detector position, or at nearly the same detector position, wherein it may be practical if the detectors are configured and arranged to detect radiation from different/opposite directions. It may be practical if the radiation body comprises a slab of material, wherein the at least two radiation sources and/or the at least two detectors are embedded in the slab of material. The material of the slab of material may be silicone, for example. The at least one radiation source may be an LED, particularly a UV-C LED, for example. In such a case, the at least one detector may comprise the same LED, for example, in view of the fact that it is possible to use a LED as a radiation detector, wherein it is practical if the controller arrangement is configured to put the LED to a detector state instead of a radiation source state from time to time, or the at least one detector may comprise a separate radiation detector of any suitable type.

The invention also relates to an object comprising the system as described here before, wherein the radiation exit window of the radiation body of the system is arranged to be at an exterior side of the object. Examples of such an object include all of the examples mentioned in the foregoing in respect of the system known from WO 2018/215272 A1. In general, the objects may be selected from a group comprising domestic appliances, furniture, construction elements of buildings and vehicles, sanitary parts, medical devices, and components of all of the foregoing. The object may be a door knob, control button, touchscreen or the like that is especially intended to be touched regularly and temporarily by human beings, particularly different human beings.

The invention also relates to a method of adding a system as described here before to an existing object, wherein the radiation body of the system is applied to an exterior surface of the existing object. This may be done in any suitable way, including through gluing, by using fastening means or on the basis of a snap connection or form closure arrangement.

The above-described and other aspects of the invention will be apparent from and elucidated with reference to the following detailed description of practical embodiments of a system comprising i) a radiation body comprising a radiation exit window, wherein the radiation body is configured to receive radiation that at least comprises UV radiation, and wherein the radiation exit window is capable of allowing at least part of the radiation to pass to the exterior of the radiation body, ii) at least one radiation source configured to provide the radiation, iii) at least one detector configured to detect an internal radiation intensity in the radiation body, and iv) a controller arrangement, functionally coupled to the at least one radiation source and the at least one detector.

### BRIEF DESCRIPTION OF THE DRAWINGS

The invention will now be explained in greater detail with reference to the figures, in which equal or similar parts are indicated by the same reference signs, and in which:
Fig. 1 illustrates a basic configuration of a system according to the invention,
Fig. 2 diagrammatically shows a graph representing an output signal of a detector of the system against time and a graph representing intensity of radiation radiated by a radiation source of the system against time, and
Fig. 3 diagrammatically shows a number of objects which can be equipped with the system according to the invention.

### DETAILED DESCRIPTION OF THE EMBODIMENTS

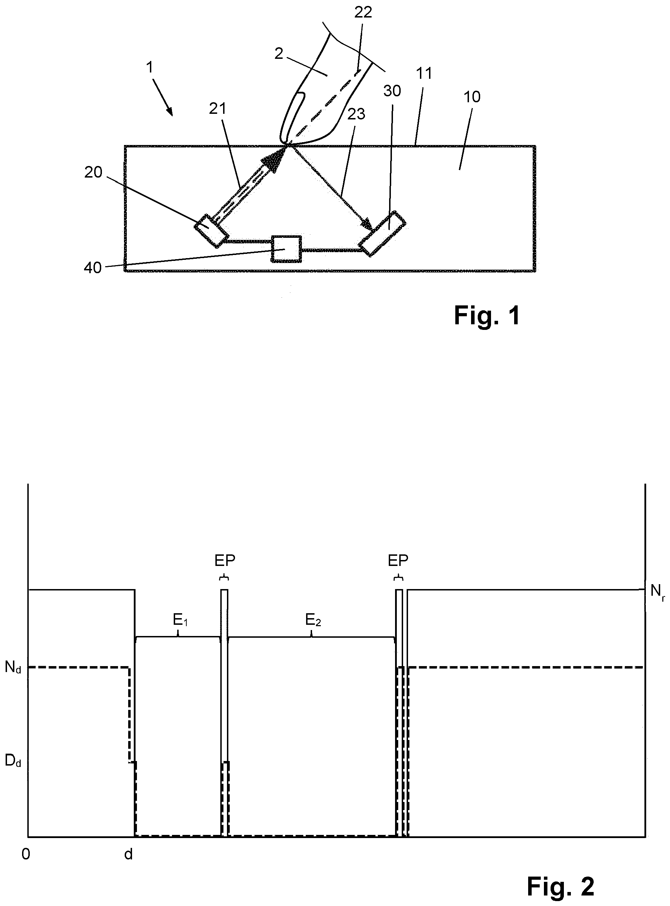

Fig. 1 illustrates a basic configuration of a system 1 according to the invention.

The system 1 comprises a radiation body 10, a radiation source 20, a detector 30, and a controller arrangement 40.

The radiation body 10 comprises a radiation exit window 11. Fig. 1 illustrates the practical options of the radiation body 10 comprising a slab of material and the radiation exit window 11 being of generally flat appearance.

The radiation source 20 is configured to emit radiation that at least comprises UV radiation, in one or more directions, and may be a UV-C LED, for example. In Fig. 1, radiation emitted by the radiation source 20 is depicted by means of an arrow 21. Further, Fig. 1 illustrates the practical option of the radiation source 20 being embedded in the slab of material of the radiation body 10, wherein the slab of material is transparent to the radiation 21 provided by the radiation source 20. Any appropriate number of radiation sources 20 can be chosen in the context of the invention, wherein it is possible to have an arrangement of the radiation sources 20 in which the radiation sources 20 are positioned to radiate radiation 21 to respective areas of the radiation exit window 11 of the radiation body 10, whether or not with overlap.

The detector 30 is configured to detect an internal radiation intensity in the radiation body 10. Fig. 1 illustrates the practical option of the detector 30 being embedded in the slab of material of the radiation body 10. Any appropriate number of detectors 30 can be chosen in the context of the invention.

The controller arrangement 40 is functionally coupled to the radiation source 20 and the detector 30 and is configured to control operation of the system 1. The invention covers both an option of the system 1 comprising a communication arrangement configured to enable data communication between the controller arrangement 40 and the radiation source 20 and the detector 30, respectively, to take place in a wired fashion and an option of the system 1 comprising a communication arrangement configured to enable the data communication to take place in a wireless fashion, and also covers an option of the system 1 comprising a communication arrangement configured to enable the data communication to take place partly in a wired fashion and partly in a wireless fashion. Fig. 1 illustrates the practical option of the controller arrangement 40 comprising a unit that is embedded in the slab of material of the radiation body 10. That does not alter the fact that it is also possible that additionally or alternatively, the controller arrangement 40 comprises at least one unit that is positioned outside of the slab of material of the radiation body 10.

The radiation source 20, the detector 30 and the controller arrangement 40 may be powered in any suitable way and may be electrically coupled to the mains or to a battery, for example.

The radiation exit window 11 of the radiation body 10 is configured to transmit part of the radiation 21 of the radiation source 20. In Fig. 1, the outcoupled radiation is indicated by means of a dashed line 22. The detector 30 is at a position of receiving radiation 23 emanating from the radiation exit window 11 inward into the slab of material of the radiation body 10, and is configured to provide an output signal to the controller arrangement 40 representing intensity of such internal radiation 23. Depending on the structure of the radiation exit window 11, the internal radiation 23 may be obtained through at least one of i) scattering of the radiation 21 radiated by the radiation source 20 at the position of the radiation exit window 11 and ii) reflection of the radiation 21 radiated by the radiation source 20 on an interior side of the radiation exit window 11. It may be so that scattering of the radiation 21 radiated by the radiation source 20 especially takes place when an object 2 such as a person's finger is present on an exterior side of the radiation exit window 11, as diagrammatically shown in Fig. 1. In any case, when the radiation exit window 11 is contacted by an object 2, both the outcoupled radiation 22 and the internal radiation 23 will likely change.

The main purpose of having the radiation source 20 in the system 1 is keeping the exterior side of the radiation exit window 11 in a disinfected state. When a bacteria or a virus ends up on the exterior side of the radiation exit window 11, the bacteria or virus is killed or at least rendered inactive under the influence of the outcoupled radiation 22 at the position of the bacteria or virus. In that way, spread of diseases via the radiation exit window 11 is prevented. This functionality of the system 1 is advantageous in many possible applications of the system 1, particularly applications in which the radiation exit window 11 is prone to be regularly and temporarily touched by human beings and/or animals/pets.

The controller arrangement 40 is configured to control the radiation source 20 in dependence of the signal provided by the detector 30, especially when it comes to setting the intensity of the radiation 21 emitted by the radiation source 20. The fact is that for safety reasons, it is desired to reduce the intensity of the radiation 21, probably to zero, in a situation of a person or animal/pet touching the radiation exit window 11. In view thereof, the controller arrangement 40 is configured to set a normal state of the radiation source 20 in which the radiation source 20 provides the radiation 21 with an intensity at an operational level as a default, and to set an adapted state of the radiation source 20 in which the radiation source 20 provides the radiation 21 with an intensity at an adapted level that is reduced relative to the operational level after occurrence of a disturbance incident involving a change of the internal radiation intensity detected by the at least one detector 30. In this respect, it is noted that occurrence of a disturbance incident may be determined when a value of the internal radiation intensity is outside of a safety reference range of values and/or when a change of the internal radiation intensity in predetermined direction takes place at a rate that is higher than a disturbance reference rate.

According to a notable aspect of the invention, the controller arrangement 40 is configured to apply a safety algorithm after occurrence of a disturbance incident. With reference to Fig. 2, the nature of the safety algorithm will now be explained. In the figure, a graph representing the output signal of the detector 30 against time and a graph representing intensity of the radiation 21 radiated by the radiation source 20 against time are shown. The first graph is depicted in a dashed line while the second graph is depicted in a continuous line. It is to be noted that the graphs serve for illustration purposes only and should not be regarded as being representative of possible actual values, wherein the graphs are simplified, do not show system delays, etc. During a first period of time, starting at 0, the output signal of the detector 30, which will hereinafter be referred to as the signal, is at a normal level N_{d}, and the intensity of the radiation 21 radiated by the radiation source 20, which will hereinafter be referred to as the intensity, is at a normal level Nᵣ. At a moment in time d, the signal drops to a level D_{d}. The change of the signal is such that the controller arrangement 40 determines occurrence of a disturbance incident and initiates the safety algorithm.

As a first step of the safety algorithm, the controller arrangement 40 puts the radiation source 20 to the adapted state, which in the present example involves an intensity of the radiation 21 provided by the radiation source 20 of zero, i.e. no radiation 21 at all. Consequently, the signal drops to zero as well. After a first evaluation interval E₁, the controller arrangement 40 puts the radiation source 20 to the normal state for a short evaluation period EP only and assesses the signal during that period. In the shown example, the signal still appears to be at the reduced level D_{d} when the radiation 21 is provided with the normal intensity. This means that it is still not safe to restore the normal state of the radiation source 20. After a second evaluation interval E₂, the action of putting the radiation source 20 to the normal state for a short evaluation period EP only and assessing the signal during that period is repeated. In the shown example, the signal appears to be at the normal level N_{d} again when the radiation 21 is provided with the normal intensity. This means that it is safe to restore the normal state of the radiation source 20 and that the safety algorithm can be aborted. In general, as long as it follows from an evaluation action that the signal is still at the reduced level D_{d} when the radiation 21 is provided with the normal intensity, one or more subsequent evaluation actions are to be performed until it appears that the signal is at the normal level N_{d} again.

The above-described procedure can be performed in a similar manner when the radiation source 20 is put to another state than the normal state during the evaluation period(s) EP, wherein such other state is a state in which the radiation source 20 provides the radiation 21 with an intensity at a level that is between the operational level and the adapted level.

The above-described procedure is performed all over again in respect of subsequent disturbance incidents. In order to ensure proper functioning of the system 1, the references relied upon in determining occurrence of a disturbance incident are preferably chosen such that the safety algorithm is not initiated by the presence of relatively small objects on the radiation exit window 11, particularly objects having dimensions of tens to hundreds micrometer or even smaller dimensions, such as tiny droplets and/or grease/dirt containing bacteria and/or viruses, but only when relatively large objects are present on the radiation exit window 11. In a sophisticated embodiment of the system 1, the controller arrangement 40 is configured to enable adjustment of the safety reference range of values of the internal radiation intensity, i.e. the values represented by the signal. This may be done on the basis of the signal as found in a period directly prior to occurrence of a disturbance incident or by regularly performing a calibration action at appropriate times. In any case, the invention provides an uncomplicated and cost-effective way to combine the disinfection goal with a detection of possible presence of a higher organism, as a result of which the time that the system 1 is operated with the radiation source 20 in the default state is maximized and the risk of exposure of a higher organism to the UV radiation is minimized.

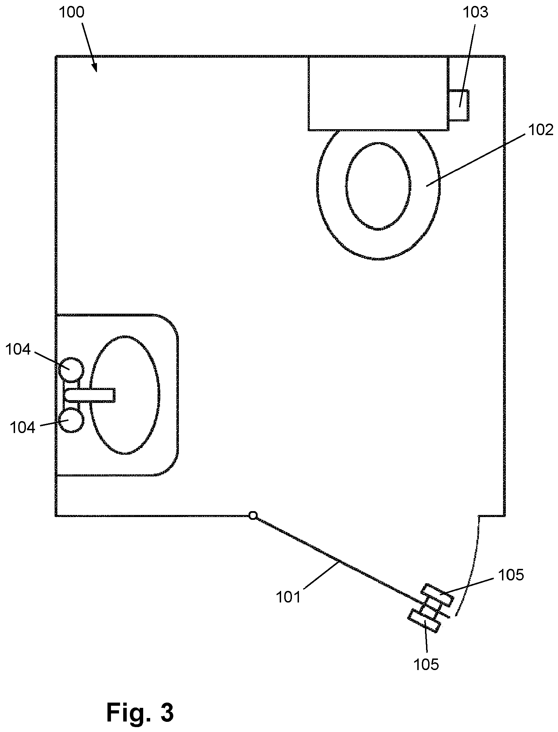

Fig. 3 diagrammatically shows a number of objects which can be equipped with the system 1 according to the invention, particularly objects as can be found in a bathroom 100 and on a bathroom door 101, namely a toilet seat 102, a toilet flushing knob 103, tap knobs 104 and the door knobs 105 of the bathroom door. The shown objects are only a few of the many objects included in the scope of protection of the invention. The object 102, 103, 104, 105 can be of conventional design, in which case the system 1 or at least the radiation body 10 of the system 1 and components arranged in the radiation body 10 can be arranged on an exterior surface of the object 102, 103, 104, 105, or the object 102, 103, 104, 105 can be of adapted design, in which case the system 1 or at least the radiation body 10 of the system 1 and components arranged in the radiation body 10 can have a sunk arrangement in a member of the object 102, 103, 104, 105, for example, wherein the radiation exit window 11 of the radiation body 10 of the system 1 can be flush with a surrounding part of an original exterior surface of the object 102, 103, 104, 105.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims or the equivalents thereof. While the invention has been illustrated and described in detail in the figures and the description, such illustration and description are to be considered illustrative or exemplary only, and not restrictive. The invention is not limited to the disclosed embodiments. The drawings are schematic, wherein details which are not required for understanding the invention may have been omitted, and not necessarily to scale.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The terms "comprise" and "include" as used in this text will be understood by a person skilled in the art as covering the term "consist of'. Hence, the term "comprise" or "include" may in respect of an embodiment mean "consist of', but may in another embodiment mean "contain/have/be equipped with at least the defined species and optionally one or more other species".

Notable aspects of the invention are summarized as follows. In a system 1 comprising a radiation body 10, at least one radiation source 20 configured to provide radiation 21 at least comprising UV radiation to the radiation body 10, at least one detector 30 configured to detect an internal radiation intensity in the radiation body 10, and a controller arrangement 40, the controller arrangement 40 is configured to apply a safety algorithm after occurrence of a disturbance incident caused by an object 2 contacting a radiation exit window 11 of the radiation body 10. The safety algorithm involves setting an evaluation state of the at least one radiation source 20 during evaluation periods separated by evaluation intervals while otherwise setting an adapted state of the at least one radiation source 20, and determining a value of the internal radiation intensity during the evaluation periods and determining a value of the internal radiation intensity during the evaluation periods in order to assess whether it is safe to restore the normal state of the at least one radiation source 20, wherein the controller arrangement 40 is configured to abort the safety algorithm and to restore the normal state of the at least one radiation source 20, indeed, when the value of the internal radiation intensity is found to be inside an evaluation reference range of values.

## Claims

1. A system (1) comprising:
- a radiation body (10) comprising a radiation exit window (11), wherein the radiation body (10) is configured to receive radiation (21) that at least comprises UV radiation, and wherein the radiation exit window (11) is capable of allowing at least part of the radiation (21) to pass to the exterior of the radiation body (10);
- at least one radiation source (20) configured to provide the radiation (21);
- at least one detector (30) configured to detect an internal radiation intensity in the radiation body (10); and
- a controller arrangement (40), functionally coupled to the at least one radiation source (20) and the at least one detector (30),
configured to set a normal state of the at least one radiation source (20) in which the at least one radiation source (20) provides the radiation (21) with an intensity at an operational level as a default, and to set an adapted state of the at least one radiation source (20) in which the at least one radiation source (20) provides the radiation (21) with an intensity at an adapted level that is reduced relative to the operational level after occurrence of a disturbance incident involving a change of the internal radiation intensity detected by the at least one detector (30), and
configured to apply a safety algorithm after occurrence of a disturbance incident that involves setting an evaluation state of the at least one radiation source (20) during evaluation periods (EP) separated by evaluation intervals (E₁, E₂), in which evaluation state the at least one radiation source (20) provides the radiation (21) with an intensity at an evaluation level that is increased relative to the adapted level, and setting the adapted state of the at least one radiation source (20) outside of the evaluation periods (EP), and that involves determining a value of the internal radiation intensity during the evaluation periods (EP), and to abort the safety algorithm and to restore the normal state of the at least one radiation source (20) when the value of the internal radiation intensity is inside an evaluation reference range of values.

2. The system (1) according to claim 1, wherein the controller arrangement (40) is configured to determine occurrence of a disturbance incident when a value of the internal radiation intensity is outside of a safety reference range of values and/or a change in predetermined direction of the internal radiation intensity takes place at a rate that is higher than a disturbance reference rate.

3. The system (1) according to claim 2, wherein the controller arrangement (40) is configured to enable adjustment of the safety reference range of values.

4. The system (1) according to claim 3, wherein the safety algorithm includes a step of adjusting the safety reference range of values on the basis of a preceding value of the internal radiation intensity detected during a period directly prior to occurrence of a disturbance incident so as to include the preceding value and a tolerance window.

5. The system (1) according to claim 3, wherein the controller arrangement (40) is configured to apply a calibration algorithm that involves determining a calibration value of the internal radiation intensity during calibration periods separated by calibration intervals, and that involves adjusting the safety reference range of values on the basis of the calibration value of the internal radiation intensity so as to include the calibration value and a tolerance window.

6. The system (1) according to claim 5, wherein the calibration intervals are of equal duration.

7. The system (1) according to any of claims 1-6, wherein the safety algorithm includes increasingly longer duration of successive evaluation intervals (E₁, E₂).

8. The system (1) according to any of claims 1-7, wherein each of the evaluation periods (EP) is at least a thousand times shorter than the evaluation interval (E₁, E₂) preceding the evaluation period (EP).

9. The system (1) according to any of claims 1-8, wherein the evaluation level of the intensity of the radiation (21) provided by the at least one radiation source (20) in the evaluation state between the adapted level and the operational level.

10. The system (1) according to any of claims 1-9, wherein the adapted level of the intensity of the radiation (21) provided by the at least one radiation source (20) in the adapted state is a zero level.

11. The system (1) according to any of claims 1-10, comprising at least two radiation sources (20) arranged in the radiation body (10) at a distance from each other and/or at least two detectors (30) arranged in the radiation body (10) at a distance from each other.

12. The system (1) according to claim 11, wherein the radiation body (10) comprises a slab of material, and wherein the at least two radiation sources (20) and/or the at least two detectors (30) are embedded in the slab of material.

13. An object (102, 103, 104, 105) comprising the system (1) according to any of claims 1-12, wherein the radiation exit window (11) of the radiation body (10) of the system (1) is arranged to be at an exterior side of the object (102, 103, 104, 105).

14. The object (102, 103, 104, 105) according to claim 13, selected from a group comprising domestic appliances, furniture, construction elements of buildings and vehicles, sanitary parts, medical devices, and components of all of the foregoing.

15. Method of adding a system according to any of claims 1-12 to an existing object (102, 103, 104, 105), wherein the radiation body (10) of the system (1) is applied to an exterior surface of the existing object (102, 103, 104, 105).
